# EUROPEAN PATENT SPECIFICATION

(11) **EP 1 461 063 B1**
(45) Date of publication and mention of the grant of the patent: **14.03.2007**
(21) Application number: 02804755.3
(22) Date of filing: 10.12.2002
(51) Int. Cl.: A61K 38/00, A61P 35/00

(54) **TREATMENT OF GLIOBLASTOMA WITH THYMOSIN-ALPHA 1**
GLIOBLASTOM-BEHANDLUNG MIT THYMOSIN-ALPHA 1
TRAITEMENT DU GLIOBLASTOME PAR LA THYMOSINE ALPHA 1

(30) Priority: 10.12.2001 US 337149 P
(43) Date of publication of application: 29.09.2004
(73) Proprietor: RHODE ISLAND HOSPITAL, Providence, RI 02903 (US)
(72) Inventor: WANDS, Jack, R., Waban, MA 02168 (US); DE LA MONTE, Suzanne, East Greenwich, RI 02818 (US)
(74) Representative: von Füner, Nicolai
(86) International application number: PCT/US2002/039329
(87) International publication number: WO 2003/049697

(56) References cited:
- EP-A- 0 433 765
- WO-A-00/69413
- WO-A-01/82949
- JEFFREY L. ROSSIO ET ALL.: "Immunotherapy of cancer with thymosin" WORLD JOURNAL OF SURGERY, vol. 1, no. 5, September 1977 (1977-09), pages 605-616, XP008049627
- GARACI ET AL.: 'Thymosin alpha 1 in the treatment of cancer: from basic research to clinical application' INT. J. IMMUNOPHARMACOL. vol. 22, 2000, pages 1067 - 1076, XP002966998

## Description

### RELATED APPLICATION DATA

This application claims the benefit of provisional application 60/337,149, filed December 10, 2001.

### BACKGROUND OF THE INVENTION

Glioblastoma is the most common primary CNS malignant neoplasm in adults, and accounts for nearly 75% of the cases. Although there has been steady progress in their treatment due to improvements in neuro-imaging, microsurgery and radiation, glioblastomas remain incurable (McDonald, 2001; Burton, 2000; Prados, 2000). The average life expectancy is less than one year from diagnosis, and the five-year survival rate following aggressive therapy including gross tumor resection is less than 10% (Burton, 2000; Nieder, 2000; Napolitano, 1999; Dazzi, 2000). Glioblastomas cause death due to rapid, aggressive, and infiltrative growth in the brain. The infiltrative growth pattern is responsible for the un-resectable nature of these tumors. Glioblastomas are also relatively resistant to radiation and chemotherapy, and therefore posttreatment recurrence rates are high. In addition, the immune response to the neoplastic cells is mainly ineffective in completely eradicating residual neoplastic cells following resection and radiation therapy (Roth, 1999; Dix, 1999; Sablotzki, 2000).

Malignant glioma cells evade detection by the host's immune system by producing immunosuppressive peptides that impair T-cell proliferation and production of IL-2 (Dix, 1999). The CNS is also somewhat immunoprivileged which allows malignant neoplastic cells to grow undetected. The search for effective treatment of glioblastomas in patients still continues today. Immunotherapy, or treatment via recruitment of the immune system, to fight these neoplastic cells has been researched in many models. Thymosin fraction 5 (TF5), thymosin α-1 (thymalfasin), IFN-α, and IL-2 are among the many immune-related components that have been studied for their abilities to fight-malignant neoplasms.

Carmustine (bischloroethyl nitrosurea, BCNU or BiCNU) is a chemotherapy agent in the chloroethylnitrosourea family, which includes other chemotherapeutic agents such as chlorozoticn (DCNU) (Anderson, 1975), lomustine (CCNU) (Carter, 1968), nimustine (Saijo, 1980) and ranimustine (Sekido, 1979). Chloroethylnitrosureas have been utilized as a single treatment chemotherapy for many years on primary brain tumors; however, the historical statistics do not always appear to support the effectiveness of these compounds as a single agent on brain tumors (e.g., Aquafedda, et al.). The combination of carmustine plus radiotherapy produced a modest benefit in long-term (18-month) survival in patients afflicted with malignant glioblastoma as compared with radiotherapy alone, although the difference between survival curves was not significant at the 0.05 level (Walker, 1980).

Thymosin α-1 (thymalfasin) is a 28-amino acid peptide, a synthetic form of a naturally occurring compound that is found in circulation (Bodey, 2000; Bodey, 2001). Thymalfasin stimulates thymocyte growth and differentiation, production of IL-2, T cell IL-2 receptors, 1FN-γ and IFN-α (Andreone, 2001; Sztein, 1989; Knutsen, 1999; Spangelo, 2000; Tijerina, 1997; Garbin, 1997; Attia, 1993; Cordero, 1992; Baxevamis, 1994 & 1990; Beuth, 2000). Thymalfasin has been used in clinical trials to treat hepatitis B virus infection (Chan, L-Y, 2001), hepatitis C infection (Chan, H.L., 2001; Sherman, 1998; Schinazi), carcinomas of the lung or head and neck, melanoma (Bodey, 2000 & 2001; Garaci, 2000), and AIDS (Billich, 2002). The promising results of these investigations, combined with the evidence of reduced T cell responsiveness to glioblastomas, led to the present work evaluating the potential therapeutic benefit of thymalfasin immunotherapy for treating malignant gliomas, and determining the mechanisms in which thymalfasin exerts its anti-neoplastic effects.

### SUMMARY OF THE INVENTION

The present invention concerns the use of chloroethylnitrosurea in combination with a thymosin-d1 (TA1) peptide for the manufacture of a medicament for the treatment of glioblastoma. Glioblastomas are high-grade, malignant central nervous system (CNS) neoplasms that are nearly always fatal within 12 months of diagnosis. Recent studies showed that immunotherapy using pro-inflammatory cytokines such as IL-2 or IL-12 may prolong survival of patients with glioblastomas. Thymosin-α-1 (thymalfasin) is a thymic peptide that acts as an immune-modulator, increasing IL-2 production and T-cell proliferation. The present work demonstrated significantly reduced tumor burden and increased lympho-mononuclear inflammatory cell response in subjects treated with thymalfasin+BCNU relative to all other groups. In vitro experiments demonstrated that thymalfasin treatment had no direct effect on viability or mitochondrial function in cultured 9L cells. However, thymalfasin treatment resulted in significantly increased levels of pro-apoptosis gene expression, including FasL, FasR and TNFα-IR (65.89%, 44.08% and 22.18%, respectively). In addition, thymalfasin treatment rendered the 9L cells more sensitive to oxidative stress such that ordinarily non-lethal doses of H₂O₂ killed 30-50% of 9L cells that had been treated with thymalfasin. Further studies revealed that thymalfasin enhances 9L cell sensitivity to Granzyme B- (T cell) or BCNU-mediated killing. The results show that thymalfasin enhances chloroehtylnitrosurea-mediated eradication of glioblastoma in vivo, and that thymalfasin mediates its effects by activating pro-apoptosis mechanisms, rendering neoplastic cells more sensitive to oxidative stress and killing by Granzyme B (T cells) or chemotherapy.

### BRIEF DESCRITPION OF THE FIGURES

Figure 1 shows that thymalfasin has minimal effect on 9L cell viability and mitochondrial function.

Figure 2 shows the increased pro-apoptosis gene expression in 9L cells exposed to thymalfasin for 72 hours.

Figure 3 shows that thymalfasin (THY) renders 9L glioblastoma cells more sensitive to killing by oxidative stress or BCNU chemotherapy.

Figure 4 shows that thymalfasin renders 9L glioblastoma cells more sensitive to Granzyme B-mediated killing; panel A shows effect on cells exposed to vehicle or thymalfasin (24 hrs-acute, 72 hrs-chronic), then divided for an additional 1 hour treatment with vehicle, and panel (B) shows effect on cells exposed to vehicle or thymalfasin (24 hrs-acute, 72 hrs-chronic), then divided for an additional 3 hours treatment with vehicle.

Figure 5 shows the time course development of clinico-neruopathological abnormalities following implantation of 10,000 9L glioblastoma cells into the right frontal lobes of adult Long Evans rats.

Figure 6 shows the effect of BCNU and BCNU + thymalfasin (THY) on glioblastoma progression in vivo.

Figure 7 shows reduced glioblastoma burden and 25% cure in rats treated with BCNU+thymalfasin (THY).

### DETAILED DESCRIPTION

It has now been found that thymalfasin can potentiate immune-mediated killing of glioblastoma cells, making its use as an adjuvant in combination with a chloroethylnitrosurea chemotherapeutic compound an effective anti-glioblastoma therapy.

The invention is applicable to thymalfasin (TA1) peptides including naturally occurring TA1 as well as synthetic TA1 and recombinant TA1 having the amino acid sequence of naturally occurring TA1, amino acid sequences substantially similar thereto, or an abbreviated sequence form thereof, and their biologically active analogs having substituted, deleted, elongated, replaced, or otherwise modified sequences which possess bioactivity substantially similar to that of TA1, e.g., a TA1 derived peptide having sufficient amino acid homology with TA1 such that it functions in substantially the same way with substantially the same activity as TA1.

The in vivo studies using an experimental model of glioblastoma demonstrated that while BCNU treatment did significantly reduce tumor burden, the response were heterogeneous with many cases exhibiting no detectable response. However, treatment with thymalfasin+BCNU provided significant therapeutic benefit both with respect to reducing mean tumor burden and curing tumors in approximately 25% of the cases. The thymalfasin+BCNU-mediated reductions in tumor burden were associated with increased lympho-mononuclear cell infiltrates within and surrounding the neoplastic cells in the brain. In cases where no residual tumor could be found, only gliotic scar tissue and scant inflammation associated with the initial tumor cell infiltrates were detected. Glioblastoma cures were observed in approximately 25% of the low-dose and high-dose thymalfasin + BCNU treated groups.

A somewhat unexpected finding was that the thymalfasin-only groups fared the same as control or worse. Frequently, the brains of thymalfasin-treated rats were more swollen and herniated due to inflammation and edema combined with the growing tumor mass. In these regards it is noteworthy that rats treated with the lower concentration of thymalfasin ± BCNU had better outcomes than those treated with the higher concentration of thymalfasin ± BCNU since the tumor cell killing was similar in the two groups, but edema and herniation were more prevalent in the group that received the higher concentration of thymalfasin.

Therefore, thymalfasin-treatment alone did not eradicate the glioblastomas, and was probably detrimental due to the excess swelling in the absence of concomitant tumor cell killing. The results further suggested that thymalfasin had little or no direct cytotoxic effects on malignant neoplastic cells, and that the additional tumor cell killing observed with thymalfasin+BCNU treatment was mediated by indirect actions of thymalfasin. In brains of thymalfasin-treated rats, the finding of increased densities of lympho-mononuclear inflammatory cells that were characterized as predominantly T cells and macrophages suggests that thymalfasin has an important role in recruiting effector immune cells to malignant neoplasms.

A series of in vitro experiments were conducted to determine the mechanism by which thymalfasin mediates its anti-glioblastoma effects. Initial studies determined that thymalfasin had no significant direct cytotoxic effects on the glioblastoma cells. The same was true for other cell types including neuroblastoma cells and post-mitotic cortical neurons. To extend these investigations, we evaluated whether thymalfasin adversely affected cell function and perhaps rendered them more susceptible to apoptosis. To do this, we examined the expression levels of pro-apoptosis and pro-survival genes, as well as growth and housekeeping genes. Those studies revealed that thymalfasin treatment for 24 or 72 hours resulted in significantly increase levels of pro-apoptosis genes in 9L glioblastoma cells. Similar results were obtained for Sy5y neuroblastoma cells. In 293 cells, the same phenomenon was noted except that pro-survival mechanisms were inhibited and the pro-apoptosis genes were unaffected. These findings suggest that although thymalfasin has no direct cytotoxic effects, it may render cells more sensitive to cytotoxic agents by increasing basal expression of pro-apoptosis genes or reducing basal expression of survival genes. To test this hypothesis, we determined if thymalfasin-treated cells were more sensitive to oxidative stress or chloroethylnitrosurea-mediated killing. The studies showed that after 24 or 72 hours of thymalfasin treatment, sublethal concentrations of H₂O₂ or BCNU respectively killed 25% or 40% of the 9L cells. Therefore, at least some of the effects of thymalfasin were mediated by its actions on the neoplastic cells rather than being entirely due to immune modulation and recruitment of T cells and macrophages.

The immune-modulating properties of thymalfasin and related molecules has been established. Its major effects are to increase pro-inflammatory cytokine production and lymphocyte proliferation. Activated T lymphocytes kill target cells through FasL-FasR interactions and by activating the perforin-granzyme system. The finding of increased FasL/FasR expression in thymalfasin-treated 9L glioblastoma cells suggests that activated T cells could effectively kill these target cells though FasL/FasR interactions. However, utilizing a novel in vitro assay constructed with SLO (permeabilizing agent) and recombinant Granzyme B instead of activated T cells, we demonstrated that thymalfasin-treated 9L glioblastoma cells were rapidly killed by exposure to SLO and Granzyme B. These findings suggest that thymalfasin may effectively promote immune-mediated killing of glioblastoma cells in several ways: 1) increasing basal levels of pro-apoptosis gene expression rendering the cells more sensitive to oxidative stress and cytotoxic/chemotherapeutic agents; 2) increasing levels of FasR which could interact with FasL on activated T cells and lead to increased apoptosis; and 3) recruiting activated T cells and macrophages and enhancing perforin-granzym mediated killing of target tumor cells. In addition, the results strongly indicate that thymalfasin treatment of glioblastomas is effective when used in combination with chloroethylnitrosureas, but not as a stand-alone agent. The results provide substantial evidence that thymalfasin administered alone may be detrimental due to increased swelling and inflammation with minimal tumor cell eradication. Therefore, the most suitable role for thymalfasin in the treatment of glioblastomas is as an adjuvant agent for boosting the host immune response and eradicating residual tumor cells that survive conventional chemotherapy.

In conclusion, the work described herein demonstrates that thymalfasin exhibits anti-glioblastoma effects that are mediated through several channels including: 1) modulation of pro-apoptosis/survival genes leading to increased tumor cell sensitivity to oxidative stress or cytoxic/chemotherepeutic agents; 2) promoting FasR-FasL-mediated immune cell killing cascades; and 3) increasing target cell sensitivity to perforin-granzyme mediated immune cell killing. However, the therapeutic effects of thymalfasin with respect to its anti-glioblastoma properties were dependent upon the concomitant administration of chloroethylnitrosureas, emphasizing that thymalfasin would be best suited as an adjuvant immune modulator rather than a definitive anti-neoplastic agent. It is also likely that when combined with other chemotherapeutic compounds, thymalfasin would have similar positive effects in helping to reduce tumor burden, progression and recurrences at significantly greater rates than currently observed with conventional chemotherapy.

The invention is applicable to native (i.e., naturally occurring) thymalfasin as well as synthetic thymalfasin and recombinant thymalfasin having the amino acid sequence of native thymosin, amino acid sequences substantially similar thereto, or an abbreviated sequence from thereof, and their biologically active analogs having substituted, deleted, elongated, replaced, or otherwise modified sequences which possess bioactivity substantially similar to that of thymalfasin.

The isolation, characterization and use of thymalfasin is described, for example, in U.S. Patent No. 4,079,127, U.S. Patent No. 4,353,821, U.S. Patent No. 4,148,788 and U.S. Patent No. 4,116,951. The amount of thymalfasin necessary to elicit the desired degree of potentiation of the chemotherapeutic effect of BCNU can be determined by routine dose-titration experiments. Thymalfasin has been found to be safe for humans when administered in doses as high as 16 mg/kg body weight/day. A preferred dose of thymalfasin is in the range of 0.001 mg/kg body weight/day to 10 mg/kg body weight/day, with a dose of about 0.02 mg/kg body weight/day being most preferred.

The chloroethylnitrosurea can be administered at a dose of about 90-250 mg/m², by injection, orally, via biodegradable wafer, or any other convenient means known in the art. It may be given as a single dose or divided into daily injections such as 75 to 100 mg/m² on two successive days. Subsequent dosage from the initial dose should be adjusted according to the hematologic response of the patient from the prior dose. Blood counts should be monitored weekly and repeat courses should not be given before 6 weeks due the hematologic toxicity is delayed and cumulative. A preferred chloroethylnitrosurea is, which can be administered at a dose of 150-200 mg/m2 intravenously every 6 weeks. BCNU can also be administered by implantation of biodegradable wafers (e.g., Gliadel, Guilford Pharmaceuticals) directly to the tumor bed. If administration is via biodegradable wafer, the co-administered thymalfasin can conveniently be combined with the BCNU directly in the wafer.

### Example 1: Thymosin-α1 treatment of 9L and 293 tumor cell lines

Tumor cell lines: Rat 9L glioblastoma cells and 293 human kidney cells were maintained in Dulbecco's Modified Eagle's Medium (DMEM) supplemented with 5% FCS, 2 mM L-glutamine, and 100 µM non-essential amino acids (Gibco-BRL, Grand Island, NY). All cell lines were kept at 37°C in a humidified atmosphere containing 5% CO₂. The cell lines were obtained from the American Type Culture Collection and were certified as free of pathogens.

Thymosin alpha 1 treatment: For acute thymalfasin treatment, cells seeded in 96-well plates at a cell density of 20,000 cells/well were treated with 10⁻⁵ M thymalfasin for 24 hours. Cells treated chronically with thymalfasin were grown in flasks and treated every 24 hours with 10⁻⁵ M thymalfasin (added to fresh medium) for the duration of the treatment (3 days). In addition, for the determination of a dose-response curve to thymalfasin for 9L cells, cells were treated with serially diluted amounts of thymalfasin, yielding thymalfasin concentrations ranging from 50 uM to 0.022 uM.

H₂O₂ was used with cells to induce oxidative stress. Cells that had been treated with thymalfasin or vehicle for 24 hours were exposed to H₂O₂ ranging in concentration from 9 µM to 1.8 mM, and then evaluated for viability and mitochondrial function using the CV and MTT assays as described. In the experiments in which serially diluted amounts of thymalfasin were administered to the cells, a constant amount of the 1.8mM H₂O₂ was used to treat the cells. For 9L cells, an H₂O₂ curve was developed in order to determine the correct H₂O₂ concentration with which to treat the cells.

MTT assays were conducted on 9L and 293 cell lines to determine the effects of thymalfasin treatment and/or H₂O₂-induced oxidative stress on the mitochondrial function of the cells. After treatment, 10 µl of MTT solution were added to each well containing 100 µl of medium. The plates were incubated with the MTT dye in the 37°C incubator for 15min-1 hour, depending on the cell type. The medium was then removed, and 200 µl of acidic Isopropanol was added to each well. The plates were shaken at room temperature until cell lysis occurred, and then read in a Packard SpectraCount^{TM} machine at 540nm.

Crystal Violet (CV) was also used to stain cells in 96-well plates. After discarding the medium, 20 µl of crystal violet was added to each well and shaken at room temperature for 10 minutes. The plates were then washed several times with warm water and dried. 100-200 µl (depending on cell density) of PBS w/1% SDS was added to each well. The plates were incubated at room temperature on a shaker until the cells were sufficiently lysed. The plates were read at 540 nm to detect differences in cell viability between the various groups tested.

Microtiter Immunocytochemical ELISA (MICE) Assays (de la Monte, 1999) were done on 9L and 293 cells. Cells were seeded in 96-well plates, treated with 10⁻⁵M thymalfasin for 24 hours, and histofixed overnight prior to analysis using MICE assay, the fixed cells were permeabilized with 0.05% saponin in Tris-buffered saline (50 mM Tris, pH 7.5, 0.9% NaCl; TBS), blocked with Superblock-TBS (Pierce, Rockford, IL), and then incubated overnight at 4°C with primary antibodies to proliferating cell nuclear antigen (PCNA), bcl-2, p21/Wwaf-1, p53, FasL, FasR, TNF-R1, or GAPDH, each diluted to 0.5-1 µg/ml in TBS containing 0.05% Tween-20 and 0.5% bovine serum albumin (TBST-BSA). Immunoreactivity was detected using horseradish peroxidase conjugated secondary antibody (Pierce, Rockford, IL) and the TMB soluble substrate (Pierce, Rockford, IL). Reactions were stopped by the addition of 1 M H₂SO₄ and absorbances were measured at 450 nm in a Spectracount microplate reader (Packard Instrument Co., Meriden, CT). Subsequently, cell density was measured by staining the adherent cells with Coomassie blue dye and measuring the absorbance of the eluted dye (de la Monte, 1999). The MICE index was the calculated ratio of TMB and Coomassie blue absorbance measured in the same culture well. Mean ± S.D. of results obtained from 16 replicate culture wells were used for inter-group statistical comparisons.

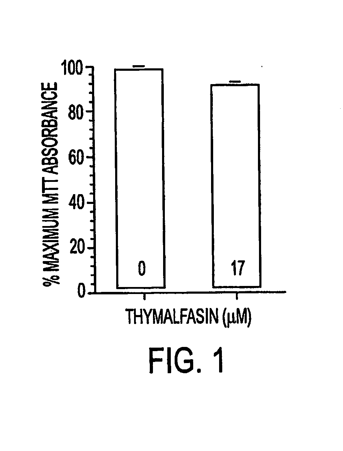

Primary experiments in which 9L cells were treated for 24 hours with various concentrations of thymalfasin showed an insignificant decrease in cell mitochondrial functioning as seen through MTT assays (Figure 1). Mitochondrial function was measured because cell death may be mediated by mitochondrial dysfunction rather than apoptosis or necrosis. The studies demonstrated similar levels of viability and MTT activity in vehicle-treated control and thymalfasin-treated cultures (Figure 1), indicating that thymalfasin does not have direct cytotoxic effects on 9L glioblastoma cells, consistent with the in vivo results. Similar results were obtained with respect to other cell lines including 293 kidney cells and SH-Sy5y neuronal cells (data not shown).

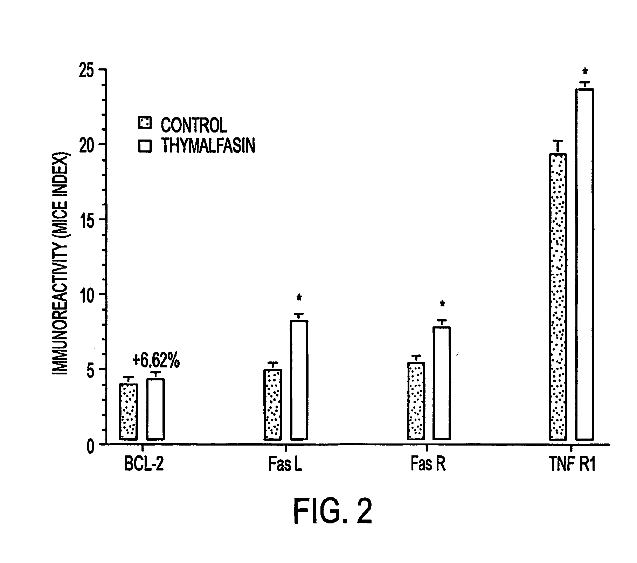

Since thymalfasin did not have direct cytotoxic effects, other potential mechanisms by which thymalfasin may function to inhibit growth of glioblastomas were explored. In this regard, the expression of gene products that promote either apoptosis or cell survival were examined, using housekeeping gene expression as control. The studies were performed in 96-well plates using the microtiter immunocytochemical ELISA (MICE) assay to generate data from multiple replicate cultures. Thymalfasin treatment for 24 hours resulted in significantly increased levels of FasR (44.08%), FasL (65.89%), and TNF-R1 (22.18%) relative to vehicle-treated controls (P<0.01; Figure 2). In contrast, expression levels of bcl-2 and GAPDH were not significantly affected by the thymalfasin treatment. Studies were also performed using 293 cells which demonstrated significant reductions in bcl-2 (36.67%; P<0.01), but no significant changes in the levels of FasR, FasL, or TNF-R1 expression (data not shown). Thus, the results of the MICE assays indicate that for the two cell lines, thymalfasin works via different pathways to increase the susceptibility of the cells to cell death by apoptosis.

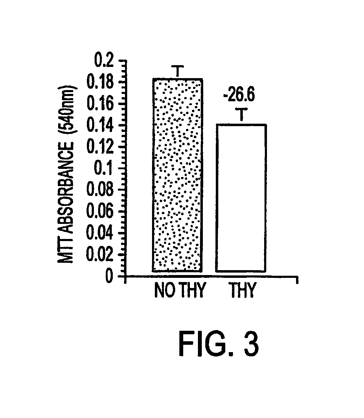

Validation of the ability of thymalfasin to increase the susceptibility of 9L and 293 cells to apoptosis was sought testing H₂O₂-induced oxidative stress. As determined through MTT assays, 9L cells treated with thymalfasin for 24 hours and subsequently H₂O₂ for 24 hours did indeed show significant loss in viability in comparison to 9L cells treated only with H₂O₂ for 24 hours (Figure 3). 9L cells treated with thymalfasin and 270 µM H₂O₂ showed a 26.6% decrease in mitochondrial functioning as seen through MTT in comparison to 9L cells treated only with 270 µM H₂O₂ (Figure 3). Similar results were obtained using 293 cells as targets in the assays (data not shown).

### Example 2: Granzyme-induced apoptosis studies

The MICE assay studies described above demonstrated thymalfasin-induced expression of TNF-R1, FasL and FasR in 9L glioblastomas. In order to determine the differential susceptibility of 9L glioblastoma cells treated (or not) with thymalfasin to cytotoxic T-lymphocyte (CTL) induced apoptosis, experimental assays were conducted in which 9L cells were treated both acutely for 24 hours and chronically for 72 hours with thymalfasin, after which they were harvested, re-seeded into 96-well black plates (7.5 x 10⁵ cells/75 µl/well), and exposed to 20,000 units/ml Streptolysin O (SLO) plus 100 ng recombinant Granzyme B (reaction volume 100 µl) for 1 or 3 hours at 37°C. The SLO was used in place of perforin to permeabilize the cells, and recombinant Granzyme B was used to standardize the assay. Control studies included parallel reactions in which SLO, Granzyme B, or both were omitted. Viable cell density was measured using the ATPlite assay (Packard Instrument Company, Meriden, CT) which has a broad linear dynamic range correlating relative light units with cell densities between 10³ to 10⁶ cells per culture well.

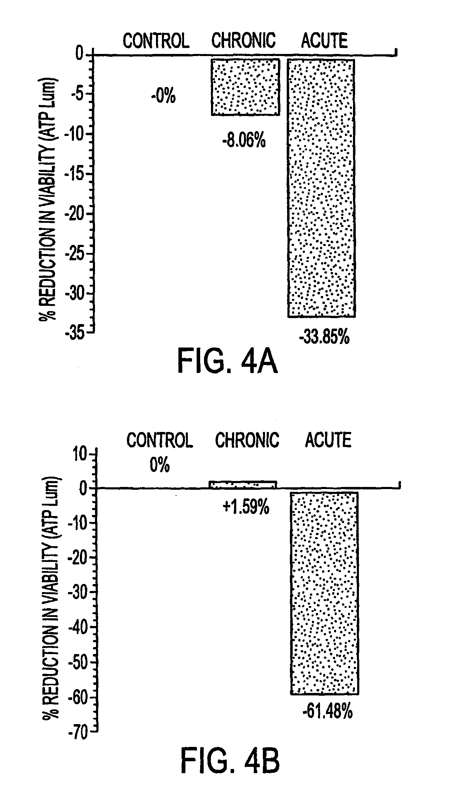

In vivo, malignant neoplastic cells are killed by cytotoxic T cells and macrophages that are recruited by pro-inflammatory cytokines such as IL-2 and IL-12. Cytotoxic T cells kill by releasing perforin which generates holes in target cell membranes, and Granzyme B which causes enzymatic destruction and death of the target cells. To study the potential role of thymalfasin in enhancing T cell-mediated killing of 9L glioblastoma cells, an in vitro assay was used in which thymalfasin- or vehicle-treated 9L cells were incubated with Granzyme B in the presence or absence of Streptolysin O (permeabilizing agent) for 1 or 3 hours. Viability was measured using the ATP luminescence assay and within group comparisons were made to determine the relative killing associated with SLO+Granzyme B treatment. The studies demonstrated significant reductions in cell viability in thymalfasin-treated cultures that were exposed to SLO+Granzyme B relative to thymalfasin-treated cultures exposed to SLO, Granzyme B or vehicle alone (p<0.001; Figures 4A and 4B). In addition, Granzyme B-mediated killing progressed over time as evidenced by the substantially higher levels of cell loss observed in assays performed after 3-hours compared with 1-hour incubation with SLO+Granzyme B (Figures 4A and 4B). In contrast, vehicle-treated control cultures exhibited similar levels of viability when exposed to SLO, Granzyme B, vehicle, or SLO+Granzyme B. In these studies, acute (24 hours) thymalfasin exposure was associated with significantly greater degrees of Granzyme B+SLO-mediated cell killing compared with cultures incubated with thymalfasin for 72 hours (chronic) prior to the assays (Figure 4).

### Example 3: Effect of thymalfasin on cell viability of primary neuronal cell cultures

Primary cortical neuron cultures were studied to enable selection of thymalfasin doses that would not be toxic to non-neoplastic brain cells. Cell viability and mitochondrial function were measured using the Crystal violet (CV) and MTT assays since previous studies showed that CV and MTT absorbances increase linearly with cell density from 1x10⁴ to 5x10⁵ cells per well (de la Monte, 2001 & 2000).

Primary neuronal cortical cells treated in 96-well plates with serial dilutions of thymalfasin (final concentrations ranging from 3.3x10⁻⁵ M to 1x10⁻⁹ M) showed no decrease in cell viability at the experimental doses used. The highest concentration of thymalfasin used (3.3 x 10⁻⁷M) did show a 30% decrease in viability as seen through MTT assay. However, this dose is higher than established experimental and clinical dosages.

### Example 4: In vivo adjuvant thymalfasin treatment of rat glioblastoma

Rat 9L glioblastoma cells were obtained from the American Type Culture Collection (Washington, D.C.) and certified as pathogen-free. The cells were maintained in Dulbecco's Modified Eagle's Medium (DMEM) supplemented with 5% fetal calf serum (FCS) and 2 mM glutamine. Antibiotics were not added to the medium. Prior to injection, the cells were rinsed with phosphate-buffered saline (PBS), detached from the culture surfaces and dissociated into single cell suspensions with 0.25% trypsin/0.05% EDTA. The dissociated cells were washed 3 times in serum-free DMEM and finally suspended in serum-free DMEM at a density of 5x10⁶ viable cells/ml. Viable cell density was determined using Trypan blue exclusion and a hemocytometer chamber.

Experiments were designed to determine if thymalfasin delivered alone or in combination with BCNU could significantly reduce glioblastoma burden compared with bischloroethyl nitrosurea (BCNU) treatment. An in vivo model of glioblastoma was generated in adult (250-300 grams) Long Evans rats. Rats were anesthetized with a single intraperitoneal injection of a cocktail containing 100 mg/kg ketamine and 100 mg/kg xylazine in 50% ethanol. After anesthetization, the head was shaved and prepped with povidone iodine, and the rat was placed in a stereotactic headframe. A midline incision was made and a 3mm burr hole was drilled over the frontal lobe, and 2µl of 9L glioma cell suspension (10,000 cells total) was injected directly into the brain using a Hamilton syringe with attached 26-gauge needle placed at a depth of 3.5 mm. After removal of the needle, the wound was washed with sterile saline, the animal was removed from the frame and the skin was closed with resorbable sutures. The rats were returned to their cages and observed for any signs of deterioration including weight loss, reduced food and water intake, hemiparesis, seizures, or inactivity. If any degree of suffering was observed, the animals were euthanized with 120 mg/kg sodium pentobarbital. Animals were observed daily and weighed weekly. Empirically, injection of 10,000 9L cells killed 100% of the animals within 26 days of implantation.

The tumor was allowed to develop for 5 days, followed by anti-neoplastic treatment. Rats were divided into 6 treatment groups: vehicle control; low (45 µg/kg) thymalfasin; high (200 µg/kg) thymalfasin; low thymalfasin + BCNU; high thymalfasin + BCNU; and BCNU (9.4 mg/kg) only. BCNU was administered as a single intraperitoneal (I.P.) injection on day 6 after the intracerebral tumor inoculation. Rats treated with thymalfasin were given single I.P. injections of thymalfasin for 3 consecutive days, beginning on day 6 after tumor cell inoculation. Rats were sacrificed on day 20 post tumor inoculation. The rats were killed by I.P. injection of 120 mg/kg sodium pentobarbital. The brains were harvested, sectioned, immersion fixed in Histochoice (Amresco Co., Solon, Ohio), and processed for paraffin embedding. Tumor burden was assessed from the gross tissue blocks and the histological sections stained with hematoxylin and eosin. Histological sections were also used in immunohistochemical staining studies to detect inflammatory cell infiltrates.

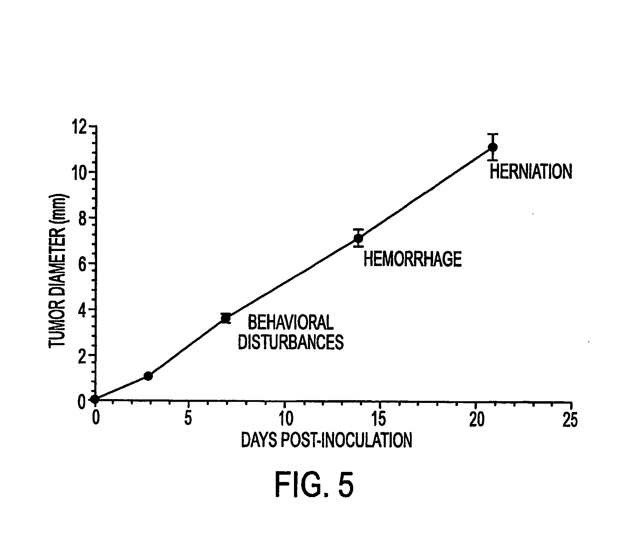

Glioblastoma Model: Intracerebral inoculation of adult Long Evans rats with 10,000 9L rat glioblastoma cells reproducibly produced tumors that progressively enlarged and caused death within 21 days. The time-dependent progression of tumor growth and associated clinical signs were characterized as follows: 1) increased physical activity with tumor diameters of 4-5 mm at day 7; 2) hyper-responsiveness by day 14 with tumor diameters of 7-8 mm and frequent associated intra-tumor hemorrhage; and 3) somnolence with tumor masses of 10-12 mm accompanied by cerebral herniation by day 21 (Figure 5). Histological sections stained with hematoxylin and eosin confirmed the presence of large tumor masses composed of infiltrative malignant neoplastic cells. Histopathological studies of coronal sections through the entire cerebrum demonstrated that within 5 days of 9L cell inoculation, the neoplastic cells formed small tumor masses that were localized in the superficial cortex and overlying leptomeninges. Within 14 days, the tumor masses extend to deeper structures including the basal ganglia and the walls of the lateral ventricle, and associated with moderate edema but no herniation (shift of midline structures). By day 21, the tumor masses occupied nearly the entire right frontal lobe with variable degrees of extension to the contra-lateral hemisphere (Figure 3). The extensive tumor mass was associated with marked cerebral edema, hemorrhage, and herniation.

A semiquantitative histological grading scale was used to assess tumor burden for inter-group comparisons: 0 - cure, no residual tumor; 1 - microscopic tumor (<1mm) confined to the superficial cortex; 2 - tumor mass occupying less than 25% of the hemisphere cross section (1-2mm); 3 - tumor mass occupying up to 50% of the hemisphere cross section (2-3mm) and extending into deep structures; 4- massive tumor burden with involvement of 50-90% of the hemisphere cross section, with herniation. The sections were coded and graded simultaneously by two separate individuals without knowledge of treatment group. To verify consistency in the grading, all samples were shuffled and re-reviewed under code.

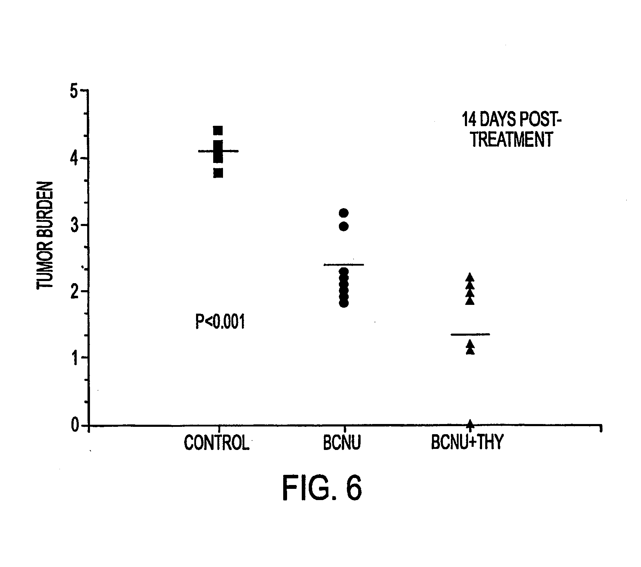

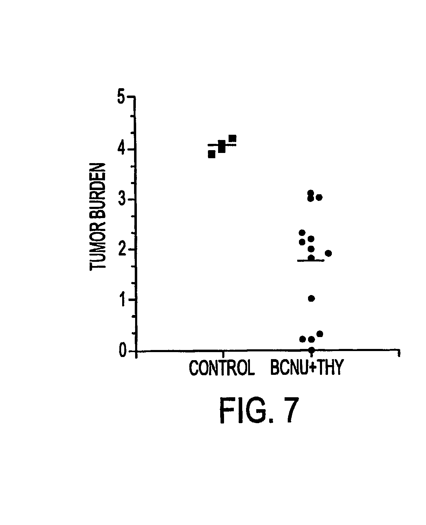

Effects of thymalfasin and BCNU on Glioblastoma Growth (Figure 6): Since spontaneous tumor rejections were not observed in preliminary studies, all experiments were terminated on day 20 after 9L glioblastoma cell implantation. Rats treated with vehicle exhibited the same time-dependent progression of tumors growth and clinical signs as observed in the untreated animals. Rats treated with BCNU exhibited significant reductions in tumor mass relative to vehicle-treated controls. However, the responses were heterogeneous with nearly half the group manifesting no apparent therapeutic response. In the remaining animals, the tumor burdens were reduced by up to 50%. Rats treated only with thymalfasin had tumor growth and clinical deterioration rates that were similar to control. In addition, the thymalfasin-treated rats had extreme intracerebral swelling and substantially greater degrees of herniation (brain tissue protruding through the Burr hole) compared with all other groups, including controls. In contrast, the thymalfasin+BCNU group exhibited the lowest gross tumor burden with gross evidence of tumor regression. Using the standardized grading scheme to assess tumor burden, we demonstrated that BCNU treatment alone significantly reduced tumor burden relative to vehicle- or thymalfasin (low or high dose)-treatment (P<0.001), and that rats treated with either low (45 µg/kg) or high (200 µg/kg) dose thymalfasin+BCNU had the lowest mean tumor burdens (Figure 6). Further studies confirmed the added clinical and pathological improvement with reduced tumor burdens plus 25% cure rates in the thymalfasin+BCNU treatment group (P<0.001; Figure 7).

Histopathological analysis also demonstrated the presence of lympho-mononuclear inflammatory cells adjacent to and within the tumor foci. In brains of vehicle-treated or BCNU-treated rats, the infiltrates were scant and mainly distributed in perivascular spaces. In the thymalfasin-treated rats, with or without BCNU, the inflammatory cell infiltrates were conspicuous and associated with the tumor masses as well as the adjacent parenchyma and overlying leptomeninges. Immunohistochemical staining studies identified the cells as T lymphocytes and macrophages. Rats treated with high-dose thymalfasin, with or without BCNU, had more cerebral swelling than rats treated with low-dose thymalfasin. Histopathological and immunohistochemical staining studies confirmed the presence of more extensive edema and inflammation associated with the tumor masses in the high thymalfasin group.

### REFERENCES

Andreone P, Cursaro C, Gramenzi A, Margotti M, Ferri E, Talarico S, (2001) "In vitro effect of thymosin alpha 1 and interferon alpha on Th1 and Th2 cytokine synthesis in patients with chronic hepatitis C" Journal of Viral Hepatitis 8: 194-201.
Aquafredda, D., Brain Tumor Treatmants: BCNU (Carmustine), http://virtualtrials.com/bcnu2.cfm
Attia, W.Y., et al., Thymosin stimulates interleukin-6 production from rat spleen cells in vitro. Immunopharmacology, 1993. **26**(2): p. 171-9.
Baxevanis, C.N., et al., Induction of lymphokine-activated killer activity in mice by prothymosin alpha. Cancer Immunol Immunother, 1994. **38**(4): p. 281-6.
Baxevanis, C.N., et al., Enhancement of human T lymphocyte function by prothymosin alpha: increased production of interleukin-2 and expression of interleukin-2 receptors in normal human peripheral blood T lymphocytes. Immunopharmacol Immunotoxicol, 1990. 12(4): p. 595-617.
Beuth, J., J.M. Schierholz, and G. Mayer, Thymosin alpha(1) application augments immune response and down-regulates tumor weight and organ colonization in BALB/c-mice. Cancer Lett, 2000. **159**(1): p. 9-13.
Billich, A., Thymosin alpha 1, SciClone Pharmaceuticals. Curr Opin Investig Drugs, 2002. **3**(5): p. 698-707.
Bodey, B., et al., Review of thymic hormones in cancer diagnosis and treatment. Int J Immunopharmacol, 2000. **22**(4): p. 261-73.
Bodey, B., Thymic hormones in cancer diagnostics and treatment. Expert Opin Biol Ther, 2001. **1**(1): p. 93-107.
Burton, E.C. and M.D. Prados, Malignant gliomas. Curr Treat Options Oncol, 2000. **1**(5): p. 459-68.
Chan, H.L., et al., The efficacy of thymosin in the treatment of chronic hepatitis B virus infection: a meta-analysis. Aliment Pharmacol Ther, 2001. **15**(12): p. 1899-905.
Chan, L-Y. (2001) "Thymosin alpha 1 for the treatment of chronic hepatitis B virus infection: a meta-analysis." Digestive Disease Week, Atlanta, Georgia, Abstract 2910
Cordero, O.J., et al., Prothymosin alpha enhances human natural killer cell cytotoxicity: role in mediating signals for NK activity. Lymphokine Cytokine Res, 1992. **11**(5): p. 277-85.
Dazzi, C., et al., A sequential chemo-radiotherapeutic treatment for patients with malignant gliomas: a phase II pilot study. Anticancer Res, 2000. **20**(1B): p. 515-8.
de la Monte, S.M., N. Ganju, and J.R. Wands, Microtiter immunocytochemical ELISA assay. Biotechniques, 1999. **26**(6): p. 1073-6, 1078.
de la Monte, S.M., et al., Oxidative stress and hypoxia-like injury cause Alzheimer-type molecular abnormalities in central nervous system neurons. Cell Mol Life Sci, 2000. **57**(10): p. 1471-81.
de la Monte, S.M., et al., Ethanol impairs insulin-stimulated mitochondrial function in cerebellar granule neurons. Cell Mol Life Sci, 2001. **58**(12-13): p. 1950-60.
Dix, A.R., et al., Immune defects observed in patients with primary malignant brain tumors. J Neuroimmunol, 1999. **100**(1-2): p. 216-32.
Garaci, E., Pica, F., Rasi, G., Palamara, A. T., and Favalli, C. Combination therapy with BRMs in cancer and infectious diseases, Mech Ageing Dev. 96: 103-16. 1997.
Garaci, E., et al., Thymosin alpha 1 in the treatment of cancer: from basic research to clinical application. Int J Immunopharmacol, 2000. **22**(12): p. 1067-76.
Garbin, F., et al., Prothymosin alpha 1 effects, in vitro, on the antitumor activity and cytokine production of blood monocytes from colorectal tumor patients. Int J Immunopharmacol, 1997. **19**(6): p. 323-32.
Knutsen, A.P., et al., Thymosin-alphal stimulates maturation of CD34+ stem cells into CD3+4+ cells in an in vitro thymic epithelia organ coculture model. Int J Immunopharmacol, 1999. **21**(1): p. 15-26.
MacDonald, D.R., Temozolomide for recurrent high-grade glioma. Semin Oncol, 2001. **28**(4 Suppl 13): p. 3-12.
Napolitano, M., et al., Treatment of supratentorial glioblastoma multiforme with radiotherapy and a combination of BCNU and tamoxifen: a phase II study. J Neurooncol, 1999. **45**(3): p. 229-35.
Nieder, C., A.L. Grosu, and M. Molls, A comparison of treatment results for recurrent malignant gliomas. Cancer Treat Rev, 2000. **26**(6): p. 397-409.
Physician's Desk Reference, 48^{th} Ed. (1994) 651-3 "BiCNU".
Prados, M.D. and V. Levin, Biology and treatment of malignant glioma. Semin Oncol, 2000. **27**(3 Suppl 6): p. 1-10.
Roth, W. and M. Weller, Chemotherapy and immunotherapy of malignant glioma: molecular mechanisms and clinical perspectives. Cell Mol Life Sci, 1999. **56**(5-6): p. 481-506.
Sablotzki, A., et al., Dysregulation of immune response following neurosurgical operations. Acta Anaesthesiol Scand, 2000. **44**(1): p. 82-7.
Schinazi, RF, Sommadossi, J-P, and Thomas, HC, editors. International Medical Press, 379-383.
Sherman, K.E. and Sherman, S.N. (1998) "Interferon plus thymosin alpha 1 treatment of chronic hepatitis C infection: a meta-analysis," in Therapies for Viral Hepatitis. Spangelo, B.L., et al., Interleukin-1 beta and thymic peptide regulation of pituitary and glial cell cytokine expression and cellular proliferation. Ann N Y Acad Sci, 2000. **917**: p. 597-607.
Sztein, M.B. and S.A. Serrate, Characterization of the immunoregulatory properties of thymosin alpha 1 on interleukin-2 production and interleukin-2 receptor expression in normal human lymphocytes. Int J Immunopharmacol, 1989. **11**(7): p. 789-800.
Tijerina, M., et al., A novel thymosin peptide stimulates interleukin-6 release from rat C6 glioma cells in vitro. Neuroimmunomodulation, 1997. **4**(3): p. 163-70.
Walker M.D., Green S.B., Byar D.P., Alexander E., Batzdorf U., Brooks W.H., Hunt W.E., MacCarty C.S., Mahaley M.S., Mealey J., Owens G., Ransohoff J., Robertson J.T., Shapiro W.R., Smith K.R., Wilson G.B., Strike TA randomized comparisons of radiotherapy and nitrosureas for the treatment of malignant glioma after surgery, NEJM, 303:1323-1329, 1980.

## Claims

1. The use of a chloroethylnitrosurea in combination with a thymosin-α1 (TA1) peptide, for the manufacture of a medicament for treating glioblastoma.

2. The use of claim 1 wherein the chlorethylnitrosurea is BCNU.

3. The use of claim 2 wherein the BCNU is administered at a dose of 150-200 mg/m².

4. The use of claim 1 wherein the (TA1) peptide is thymosin-α1, and is administered at a dose of 0.001 mg/kg body weight/day to 10 mg/kg body weight/day.

5. The use of claim 4, wherein the thymosin-α1 is administered at a dose of 0.02 mg/kg body weight/day.

## Patentansprüche

1. Verwendung von Chlorethylnitrosourea in Kombination mit einem Thymosin-α1 (TA1) Peptid bei der Herstellung eines Medikamentes zur Behandlung des Glioblastoms.

2. Verwendung nach Anspruch 1, wobei die Chlorethylnitrosourea BCNU ist.

3. Verwendung nach Anspruch 2, wobei BCNU in einer Dosis von 150-200 mg/m² verabreicht wird.

4. Verwendung nach Anspruch 1, wobei das (TA1) Peptid Thymosin-α1 ist und in einer Dosis von 0,001 mg/kg Körpergewicht/Tag bis 10 mg/kg Körpergewicht/Tag verabreicht wird.

5. Verwendung nach Anspruch 4, wobei das Thymosin-α1 in einer Dosis von 0,02 mg/kg Körpergewicht/Tag verabreicht wird.

## Revendications

1. Utilisation d'une chloroéthylnitrosourée en combinaison avec un peptide thymosin-α1 (TA1), pour la fabrication d'un médicament pour traiter le glioblastome.

2. Utilisation selon la revendication 1, dans laquelle la chloroéthylnitrosourée est la BCNU.

3. Utilisation selon la revendication 2, dans laquelle la BCNU est administrée à une dose de 150 à 200 mg/m².

4. Utilisation selon la revendication 1, dans laquelle le peptide (TA1) est la thymosin-α1, et est administré à une dose de 0,001 mg/kg de poids corporel/jour à 10 mg/kg de poids corporel/jour.

5. Utilisation selon la revendication 4, dans laquelle la thymosin-α1 est administrée à une dose de 0,02 mg/kg de poids corporel/jour.
